# EUROPÄISCHE PATENTSCHRIFT

(11) **EP 3 942 983 B1**
(45) Veröffentlichungstag und Bekanntmachung des Hinweises auf die Patenterteilung: **14.02.2024**
(21) Anmeldenummer: 20020411.3
(22) Anmeldetag: 11.09.2020
(51) Int. Cl.: A47K 17/00

(54) **DESINFEKTIONSVORRICHTUNG FÜR TOILETTENBÜRSTENGRIFFE**
DEVICE FOR DISINFECTING TOILET BRUSH HANDLES
DISPOSITIF DE DÉSINFECTION POUR POIGNÉES DE BROSSE DE TOILETTE

(30) Priorität: 22.07.2020 CH 9122020
(43) Veröffentlichungstag der Anmeldung: 26.01.2022
(73) Patentinhaber: Bezold, Uwe, 8330 Pfäffikon ZH (CH)
(72) Erfinder: Bezold, Uwe, 8330 Pfäffikon ZH (CH)

(56) Entgegenhaltungen:
- WO-A1-2019/086598
- DE-A1- 10 026 522
- DE-A1-102011 109 003
- DE-U1-202008 001 955
- DE-U1-202012 006 412

## Beschreibung

### Stand der Technik - Patentrecherche

Aus DE2020100010910U1 ist eine Vorrichtung zur Bereitstellung einer Toilettenbürste mit desinfiziertem Griff bekannt. Diese Vorrichtung enthält ein System, das den Griff der Toilettenbürste beim Zurückstellen derselben genau ausrichtet, so dass danach eine den Griff umgreifende Desinfektionskammer, die mit Hilfe einer Gewindespindel am Griff auf- und abführbar ist, den Griff desinfiziert. Die Konstruktion ist in dieser Art sehr komplex, teuer und auch störungsanfällig, da sie von einer sehr exakten Ausrichtung des Bürstengriffes abhängt. Auch muss die Gewindespindel bewegt bzw. gedreht werden, was durch händische Bedienung das eigentliche Problem nur vom Bürstengriff auf den Spindelgriff verlagert, und bei elektrischer Variante einen entsprechend hohen Aufwand darstellt. Ferner kann die Toilettenbürste nur durch passgenaue Toilettenbürsten ersetzt werden, wenn ein Austausch erforderlich ist. Schließlich hat die Desinfektionskammer in dieser Form ein nur verhältnismässig kleines Volumen und bedarf daher einer eher hohen Nachfüllfrequenz.

Aus DE102011057086B3 ist ein Formkörper zur Aufnahme eines Toilettenbürstenstiels sowie eine Vorrichtung zur Bereitstellung des Formkörpers bekannt. Bei dieser Vorgehensweise erfolgt keine Reinigung oder Desinfektion, sondern eine Abdeckung der infizierten Oberfläche. Da die Formkörper nach jeder Nutzung im Abfall entsorgt werden, entsteht so ein erhöhtes Müllaufkommen, während das Objekt WC-Bürstenstiel in infiziertem Zustand zurückbleibt. Dieser Effekt kann mit weniger Müll auch durch ein Stück ohnehin vorhandenes Toilettenpapier erzielt werden.

Aus DE2020120064412U1 ist eine Halterung für eine WC-Bürste mit einer Vorrichtung zur Reinigung und/oder Desinfektion des WC-Bürstengriffs bekannt, die stationäre Düsen zum Versprühen oder Vernebeln von Reinigungs- und/oder Desinfektionsmittel im Bereich des WC-Bürstengriffs aufweist. Jedoch stellt das blosse Besprühen eines Gegenstandes mit geeignetem Mittel weder eine ausreichende Reinigung noch eine vollzogene Desinfektion dar. Darüber hinaus gibt es praktisch keinen geeigneten Abstand der Düsen zum Bürstengriff: ist er gross genug für ein komfortables Umgreifen des WC-Bürstenstiels zum Entnehmen der WC-Bürste, so geht zu viel Reinigungs- und/oder Desinfektionsmittel verloren, ist er kleiner, so besteht die Gefahr einer Gehäuseberührung beim Entnehmen der WC-Bürste. Eine Mittelverteilung durch Besprühen stellt an sich eine eher verschwenderische Variante der Desinfektionsmittelnutzung dar. Es ist generell anzuzweifeln, dass durch eine solche Besprühung die komplette Oberfläche des Bürstengriffes ohne dessen Drehung überhaupt erreicht werden kann, da die Grundversion nach vorne offen ist. Es ist auch fraglich, an welchen 'großen Reinigungs- und/oder Desinfektionsmitteltank' die Düsen angeschlossen werden können/sollen, da die Platzverhältnisse in WC-Räumen eher begrenzt sind. Ferner ist für den Betrieb dieser Konstruktion eine Stromversorgung für Sensoren, Schalter und Pumpentechnik erforderlich, was einen hohen Aufwand in Herstellung, Installation und Wartung darstellt.

### Stand der Technik - Marktrecherche

Von denen durch die Patentrecherche ermittelten Produkte konnte trotz intensiver Suche am aktuellen Markt keines gefunden werden, obwohl der generelle Desinfektionsbedarf derzeit so gross ist, wie nie zuvor.

Am aktuellen Markt bekannt ist ein 'WC-Sauberstab', der aus einem Material hergestellt wird, welches durch einen sogenannten 'Biomaster-Schutz', der auf der Verwendung von Silber-Ionen basiert, antibakteriell auf bestimmte Erreger wirkt. An diesem Stab kommt keine Bürste zum Einsatz, sondern spezielle Reinigungseinsätze (Tipps) im Einwegverfahren, die nicht breiter sind als der Stab selbst. Diese Reinigungseinsätze müssen jedoch vor jedem Reinigungsvorgang manuell am unteren Ende des Stabes angebracht werden, was natürlicherweise eine gewisse Zurückhaltung bzw. Ablehnung beim Nutzer hervorruft. Auch erfordert das Entfernen von Fäkalienrückständen mit einem nur sehr schmalen Reinigungsgerät eine erhöhte Bereitschaft des Nutzers gegenüber dem gewohnten Reinigungsvorgang mit einer gängigen Toilettenbürste. Ferner existiert eine Hemmschwelle bezogen auf das Vertrauen darauf, dass ein Nutzgerät im Toilettenbereich ohne jegliche Reinigung stets keimfrei bleibt, worauf man sich gerade in der aktuellen Bedrohungslage eher nicht verlassen möchte, zumal der derzeit am bedrohlichsten erscheinende Erreger in der Liste derer, gegen die der Biomaster Schutz wirkt, gar nicht enthalten ist.

### Anspruch der Erfindung

Der Erfindung liegt somit der Anspruch zugrunde eine Vorrichtung zu schaffen, welche keine der aus dem aktuellen Stand der Technik bekannten Nachteile aufweist, und die es ermöglicht, jedem Nutzer mit wenig Aufwand eine Toilettenbürste mit möglichst keimfreien Griff zur Verfügung zu stellen.

### Darstellung der Erfindung

Die Erfindung besteht aus einer Desinfektionsvorrichtung für Toilettenbürstengriffe, die in eine Halterung für Toilettenbürsten integriert ist, dadurch gekennzeichnet, dass deren Bewegungsmechanik einerseits ohne händische Berührung betätigt wird, und andererseits den Toilettenbürstengriff gänzlich umschliesst und das Desinfektionsmittel direkt auf die zu desinfizierende Griff-Oberfläche aufgebracht wird.

In der Grundversion stellt die Erfindung eine durch mechanische Bauteile erweiterte und mit einem Desinfektionsmittel-Dosierungssystem ausgestattete Toilettenbürstenhalterung dar, mit welcher eine Desinfektion des Toilettenbürstengriffs möglich ist, ohne dass es dabei zu direktem Hautkontakt kommt.

Der Nutzer tritt ein in Bodennähe installiertes Pedal nach unten, wodurch eine Mechanik bewegt wird, die ein Ventil öffnet, und zwei flexible, mit einer Desinfektionsmittelzufuhr und geformten Schwammeinsätzen versehene Greifschalen zum Bürstenstiel führt, so dass sie diesen komplett umschliessen, und schliesslich mit einer Wischbewegung den Griffbereich der Toilettenbürste unter gleichzeitiger Zufuhr von Desinfektionsmittel zuverlässig desinfizieren.

Die Vorteile gegenüber dem Stand der Technik sind:
- Komplett mechanische Bauweise ohne Strombedarf
- Unverändertes Handling der Toilettenbürste durch den Nutzer, da die Mechanik oberhalb des Bürstenstiels installiert ist
- Tatsächlich umfassende Desinfektion der Stieloberfläche, da die Mechanik den Bürstenstiel so lückenlos umgreift, wie die Hand einer menschlichen Reinigungskraft
- Sparsamer Umgang mit dem Desinfektionsmittel durch Dosierung und direktes Auftragen auf die Oberfläche
- Sehr schnelle Ausführung des Desinfektionsvorgangs durch direkte Umsetzung der Pedalbewegung
- Keine 'hochexakte' Positionierung des Toilettenbürstenstiels erforderlich, da die Greifarme den Bürstenstiel 'einfangen'
- Einfaches und unkompliziertes Wiederholen des handberührungslosen Desinfektionsvorganges
- Vertrauensbildung durch unmittelbares 'Erleben' des Desinfektionsvorganges
- Einsatz aller Arten von Desinfektionsflüssigkeiten durch einfaches Ein- und Nachfüllen
- Kostengünstige Herstellung aufgrund einfach gestalteter, rein mechanischer Bauteile
- Verwendung verschiedener Toilettenbürsten durch flexible Greifkonstruktion möglich

### Aufzählung der Zeichnungen

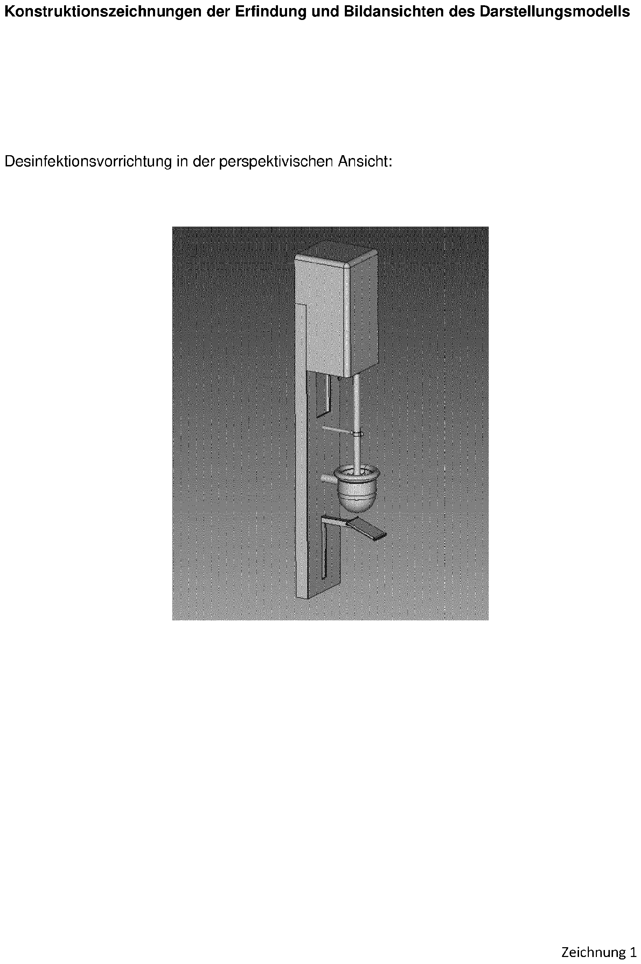

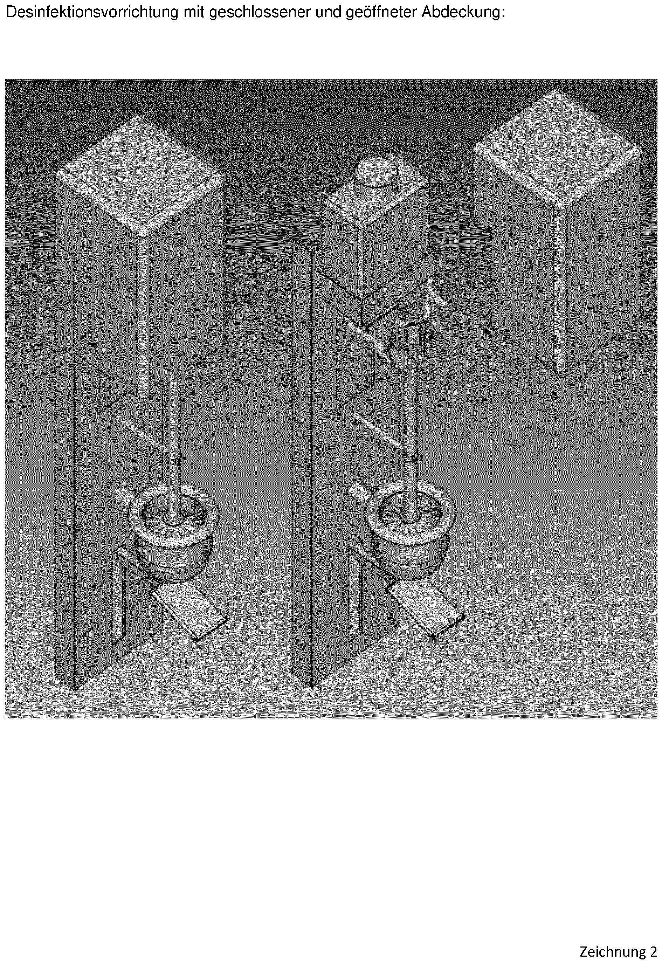

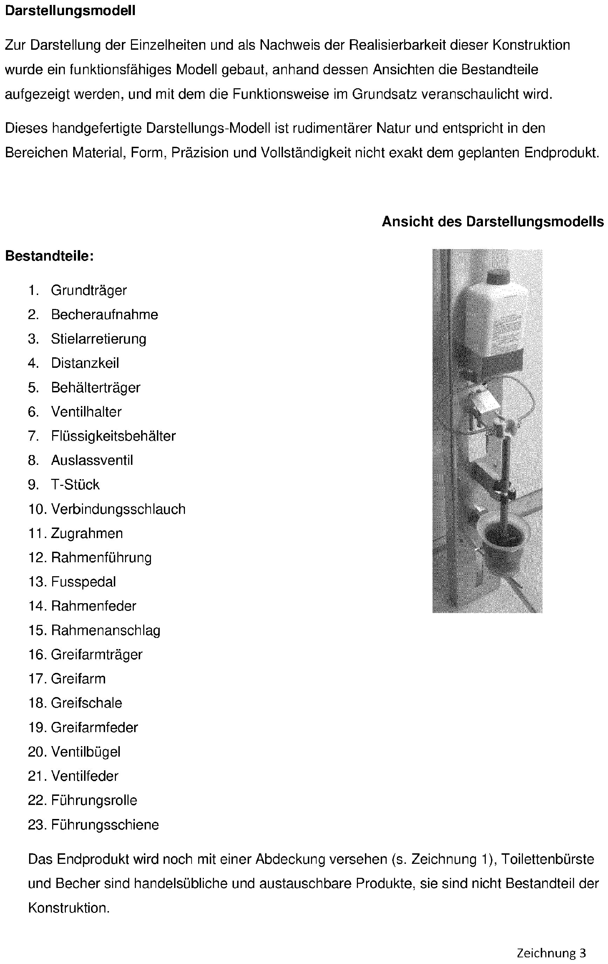

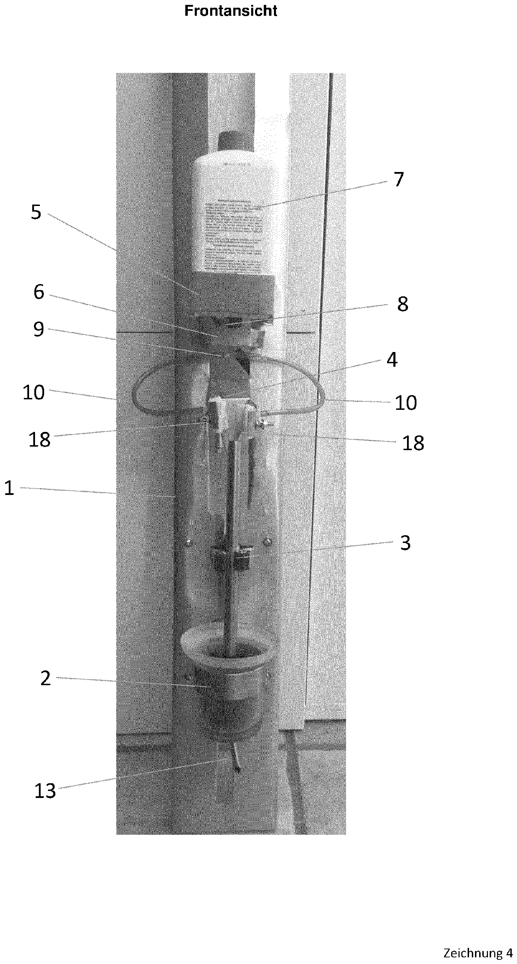

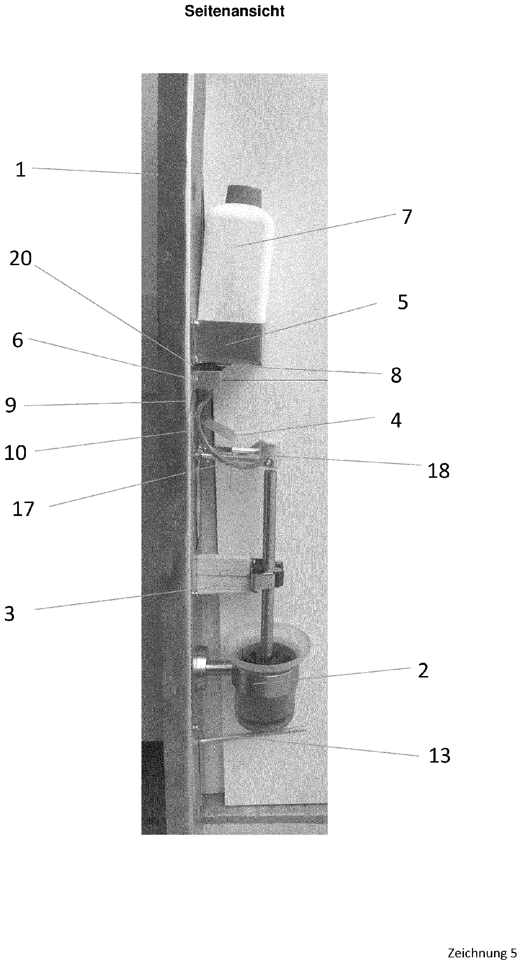

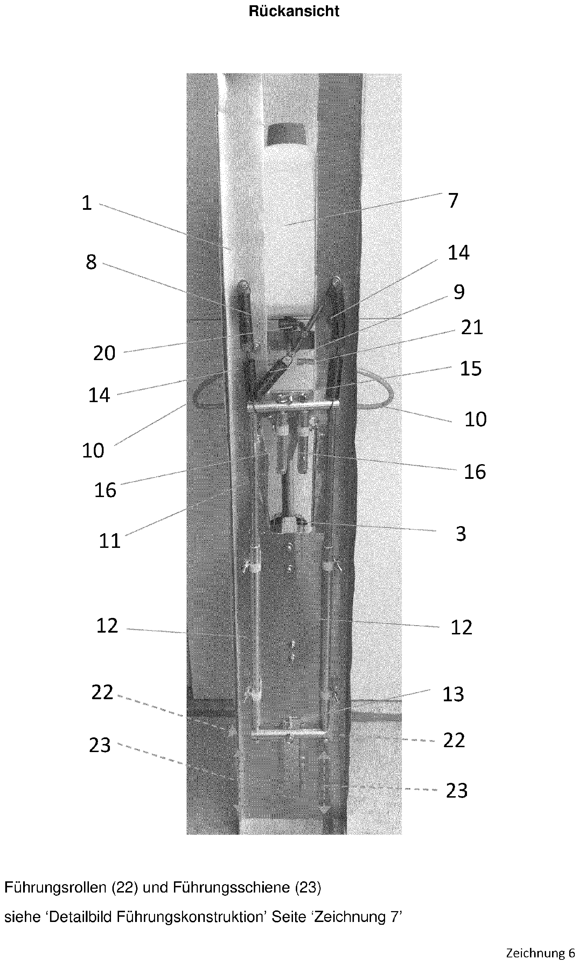

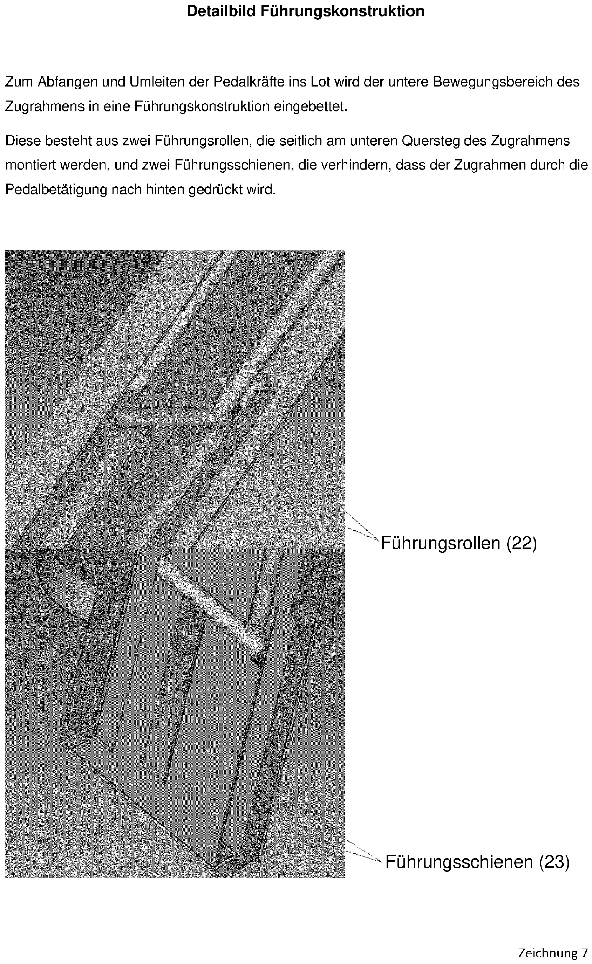

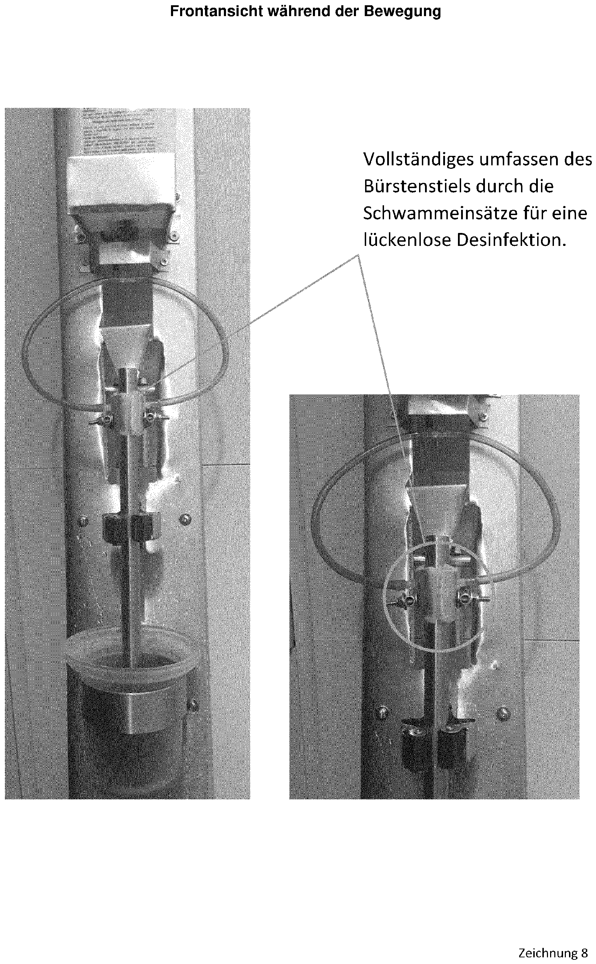

- Zeichnung 1:
   Desinfektionsvorrichtung in der perspektivischen Ansicht
- Zeichnung 2:
   Desinfektionsvorrichtung mit geschlossener und geöffneter Abdeckung
- Zeichnung 3 - Bilddarstellung:
   perspektivische Ansicht des Darstellungsmodells
- Zeichnung 4 - Bilddarstellung:
   Frontansicht mit geöffneter Abdeckung
- Zeichnung 5 - Bilddarstellung:
   Seitenansicht mit geöffneter Abdeckung
- Zeichnung 6 - Bilddarstellung:
   Rückansicht
- Zeichnung 7:
   Detailbild Führungskonstruktion
- Zeichnung 8 - Bilddarstellung:
   Frontansicht mit geöffneter Abdeckung während der Bewegung

### Ausführung der Erfindung

Die Grundversion der Erfindung wird, mit Ausnahme weniger Einzelteile, in recyclebarem Kunststoff ausgeführt.

Sie besteht im Wesentlichen aus einer Mechanik, welche mit geeigneten Bauteilen den Griff einer Toilettenbürste umfasst und die Bewegungen des Reinigungsvorgangs ausführt. Das gleichzeitige Zuführen von Desinfektionsmittel zum Bürstengriff führt zu einer zuverlässigen Desinfektion der Grifffläche.

Die Kraft der Abwärtsbewegung eines menschlichen Fusses wird über ein Trittpedal in den Mechanismus geführt, und ermöglicht so den Desinfektionsvorgang ohne einen direkten Hautkontakt und ohne weitere Energiezufuhr.

Die Einfachheit der Konstruktion lässt eine wirtschaftliche Herstellung der Vorrichtung zu, und die rein mechanische, und dadurch komplett unabhängige Funktionsweise bietet breite Einsatz- und Nutzungsmöglichkeiten.

Für die Herstellung von Einzelstücken oder einer Kleinserie dieses Produktes kann die aktuelle 3D-Drucktechnologie herangezogen werden. Dabei wird der Grundträger mit allen nicht beweglichen Teilen, die in direktem Kontakt mit ihm stehen, in einem Zuge produziert, um den erforderlichen Montageaufwand so gering wie möglich zu halten:
- Grundträger (1)
- Becheraufnahme (2)
- Stielarretierung (3)
- Distanzkeil (4)
- Behälterträger (5)
- Ventilhalter (6)
- Rahmenführung (12)
- Rahmenanschlag (15)
- Führungsschienen (23)

Der Grundträger wird ferner rückseitig einmal im oberen Drittel und einmal im unteren Drittel geschlossen, um durch diese dann teilweise vorhandene Rückwand eine Befestigungsmöglichkeit zu schaffen. Hierbei ist sowohl eine Wandbefestigung durch eine Verschraubung möglich als auch ein Anbringen durch Kleben, z. B. bei Wandfliesen.

Darüber hinaus werden im Grundträger im oberen Bereich noch zwei Haken integriert, in welche die Rahmenfedern eingehängt werden.

Die Abdeckung, deren Vorder- und Oberkanten abgerundet sind, wird mit einem Sichtschlitz versehen, um eine einfache Kontrolle des Füllstandes zu ermöglichen. Es wird von oben auf den Grundträger gesetzt, und wird durch zwei Federhaken auf der Innenseite fixiert. Dadurch kann es zum Zwecke des Nachfüllens von Desinfektionsmittel schnell und ohne Werkzeug geöffnet werden.

Folgende Bestandteile sind als solche am Markt erhältlich und müssen daher nicht zwingend in Eigenproduktion hergestellt werden:
- Flüssigkeitsbehälter (7)
- Auslassventil (8)
- T-Stück (9)
- Verbindungsschlauch (10)
- Rahmenfeder (14)
- Greifarmfeder (19)
- Ventilfeder (21)
- Führungsrolle (22)

Dagegen sind nachstehende Bestandteile nicht am Markt existent und müssen daher eigenständig produziert werden:
- Zugrahmen (11)
- Fusspedal (13)
- Greifarmträger (16)
- Greifarm (17)
- Greifarmschale (18)
- Ventilbügel (20)

Im Sinne eines möglichst niedrigen Montageaufwandes werden einige Bestandteile so konstruiert, dass eine schnelle Steck-Montage ausgeführt werden kann:
- Zugrahmen (11)
- Greifarmträger (16)
- Greifarm (17)
- Greifarmschale (18)
- Ventilbügel (20)

Hierbei wird der obere Quersteg des Zugrahmens als steckbares Einzelteil gefertigt, so dass der dann offene Zugrahmen, an dessen unteren Quersteg beidseitig die Führungsrollen angebracht werden, von unten in die Rahmenführung des Grundträgers eingeführt werden kann. Mit wenigen Handgriffen können dann nacheinander der obere Quersteg, die Rahmenfedern, die Greifarmträger, die Greifarme, die Greifarmfeder und die Greifschalen montiert werden. In einem weiteren Schritt werden die Greifschalen mit Schwammeinsätzen bestückt.

Das Fusspedal wird durch die untere Führungsöffnung des Grundträgers geführt und mittels einer Verschraubung mit dem unteren Quersteg des Zugrahmens verbunden.

Der Flüssigkeitsbehälter wird mittig an der Unterseite mit einem Loch versehen, in welches ein Anschluss des Auslassventils eingeführt und verklebt wird, und dann in den Behälterträger eingesetzt. Der zweite Anschluss des Auslassventils wird über ein kurzes Schlauchstück mit dem T-Stück verbunden. Vom T-Stück aus werden dann die beiden Verbindungsschläuche zu den Greifschalen geführt.

Abschliessend wird noch der Ventilbügel am Auslassventil angebracht, und die Ventilfedern eingehängt. Die untere Ventilfeder dient der Öffnung des Ventils bei gleichzeitigem Ausgleich des Unterschiedes zwischen der Bewegung des Zugrahmens und dem deutlich kleineren Ventilweg, die obere Ventilfeder sorgt für die Schliessung des Ventils, wenn die Rahmenfedern des Zugrahmen wieder nach oben bewegen.

Die eigentliche Produktherstellung ist hiermit abgeschlossen.

Nach der Montage des Produktes an der Wand wird dann ein Flächendesinfektionsmittel in den Flüssigkeitsbehälter eingefüllt, und die Toilettenbürste mit Becher eingesetzt. Die Grundversion der Erfindung ist ausgelegt auf eine gängige Grösse der Toilettenbürste mit einer Stiellänge von 26cm und einem Stieldurchmesser von 16mm, sowie eines Aussendurchmessers des Bürstenbechers von 86mm.

Das Produkt kann nun in dieser Ausführung verwendet werden.

Als weitere Ausführungen der Erfindung sind verschiedene Varianten möglich:
- Konventionelle Herstellung der Kunststoffteile (Formen, Gussverfahren etc.)
   Ist ab einer gewissen Stückzahl erforderlich
- Optisch ansprechende Ausführung in Blech und Metall
   Bringt einen höheren Montageaufwand mit sich
- Automatisierte Variante mit Motorantrieb
   Für Toiletten mit Stromanschluss (z. B. mit selbstreinigendem Toilettensitz)

## Patentansprüche

1. Desinfektionsvorrichtung, in eine Halterung für eine Toilettenbürste integriert, welche einen Flüssigkeitsbehälter für Desinfektionsflüssigkeiten sowie eine Mechanik enthält, wobei ein in Bodennähe installiertes Pedal durch den Nutzer nach unten zu treten ist, wodurch eine Mechanik bewegt wird, die ein Ventil öffnet, und zwei flexible mit einer Desinfektionsmittelzufuhr und geformten Schwammeinsätzen versehene Greifschalen zum Bürstenstiel führt, dass sie diesen komplett umschliessen, und schliesslich mit einer Wischbewegung den Griffbereich der Toilettenbürste unter gleichzeitiger Zufuhr von Desinfektionsmittel zuverlässig desinfizieren **dadurch gekennzeichnet dass** damit ein Desinfizieren des Toilettenbürstengriffs möglich ist ohne in direkten Hautkontakt mit dem Toilettenbürstengriff zu geraten.

2. Desinfektionsvorrichtung gemäss Anspruch 1, **dadurch gekennzeichnet, dass** die Mechanik manuell mit Hilfe eines Fusspedals bewegt wird.

3. Desinfektionsvorrichtung gemäss Anspruch 1, **dadurch gekennzeichnet, dass** die Mechanik durch einen Elektromotor bewegt wird.

4. Desinfektionsvorrichtung gemäss Anspruch 2 oder 3, **dadurch gekennzeichnet, dass** der Grundträger, die Abdeckung und die Mechanik aus Kunststoff sind.

5. Desinfektionsvorrichtung gemäss Anspruch 2 oder 3, **dadurch gekennzeichnet, dass** ein oder mehrere Bauteile mit dem 3D-Druckverfahren hergestellt sind.

6. Desinfektionsvorrichtung gemäss Anspruch 2 oder 3, **dadurch gekennzeichnet, dass** der Grundträger, die Abdeckung und die Mechanik aus Metall sind.

7. Desinfektionsvorrichtung gemäss Anspruch 2 oder 3, **dadurch gekennzeichnet, dass** ein kritischer Füllstand im Flüssigkeitsbehälter mit einem optischen Signal angezeigt wird.

8. Desinfektionsvorrichtung gemäss Anspruch 2 oder 3, **dadurch gekennzeichnet, dass** die Mechanik nur bei eingesetzter Toilettenbürste bewegt werden kann.

9. Desinfektionsvorrichtung gemäss Anspruch 2 oder 3, **dadurch gekennzeichnet, dass** die Greifkonstruktion flexibel ausgeführt ist, und damit auch unregelmässig geformte Bürstengriffe verwendet werden können.

## Claims

1. A disinfection device, integrated in a holder for a toilet brush and containing a fluid reservoir for disinfectant solutions as well as a mechanism, wherein a pedal installed near the floor is to be stepped down upon by the user, by which a mechanism is moved, which opens a valve and guides two flexible curved gripping pads equipped with a disinfectant supply and contoured sponge inserts to the brush handle such that they completely enclose it, and finally, with a wiping movement, reliably disinfect the handle area of the toilet brush with a simultaneous feed of disinfectant, wherein this enables the toilet brush handle to be disinfected without coming into direct skin contact with the toilet brush handle.

2. Disinfection device according to claim 1, wherein the mechanism is moved manually with the assistance of a foot pedal.

3. Disinfection device according to claim 1, wherein the mechanism is moved by an electric motor.

4. Disinfection device according to claim 2 or 3, wherein the base support, the cover and the mechanism are made of plastic.

5. Disinfection device according to claim 2 or 3, wherein one or more components are manufactured using the 3D printing method.

6. Disinfection device according to claim 2 or 3, wherein the base support, the cover and the mechanism are made of metal.

7. Disinfection device according to claim 2 or 3, wherein a critical filling level in the liquid container is indicated with a visual signal.

8. Disinfection device according to claim 2 or 3, wherein the mechanism can only be moved when the toilet brush is inserted.

9. Disinfection device according to claim 2 or 3, wherein the gripping structure is designed to be flexible, and thus irregularly shaped brush handles can also be used.

## Revendications

1. Dispositif de désinfection, intégré dans un support pour une brosse de toilette, qui contient un réservoir de liquide pour les liquides de désinfection ainsi qu'un mécanisme, une pédale installée près du sol devant être enfoncée par l'utilisateur,
en déplaçant un mécanisme qui ouvre une valve et guide deux coques de préhension flexibles, pourvues d'une alimentation en désinfectant et d'inserts en éponge formés, vers le manche de la brosse, de manière à ce qu'elles l'entourent complètement et désinfectent finalement de manière fiable la zone de préhension de la brosse pour toilettes avec un mouvement d'essuyage, tout en fournissant du désinfectant, **caractérisé en ce qu'**il permet de désinfecter le manche de la brosse de toilette sans entrer en contact direct avec la peau avec le manche de la brosse de toilette.

2. . Dispositif de désinfection selon la revendication 1, **caractérisé en ce que** le mécanisme est déplacé manuellement à l'aide d'une pédale.

3. . Dispositif de désinfection selon la revendication 1, **caractérisé en ce que** le mécanisme est actionné par un moteur électrique.

4. . Dispositif de désinfection selon la revendication 2 ou 3, **caractérisé en ce que** le support de base, le couvercle et le mécanisme sont en matière plastique.

5. . Dispositif de désinfection selon la revendication 2 ou 3, **caractérisé en ce qu'**un ou plusieurs composants sont fabriqués par le procédé d'impression 3D.

6. . Dispositif de désinfection selon la revendication 2 ou 3, **caractérisé en ce que** le support de base, le couvercle et le mécanisme sont en métal.

7. . Dispositif de désinfection selon la revendication 2 ou 3, **caractérisé en ce qu'**un niveau de remplissage critique dans le réservoir de liquide est indiqué par un signal optique.

8. . Dispositif de désinfection selon la revendication 2 ou 3, **caractérisé en ce que** le mécanisme ne peut être déplacé que lorsque la brosse de toilette est en place.

9. . Dispositif de désinfection selon la revendication 2 ou 3, **caractérisé en ce que** la structure de préhension est flexible et permet ainsi d'utiliser des manches de brosse de forme irrégulière.
